(19) Europäisches Patentamt
European Patent Office
Office européen des brevets

(11) **EP 2 120 682 B1**

(12) **EUROPÄISCHE PATENTSCHRIFT**

(45) Veröffentlichungstag und Bekanntmachung des Hinweises auf die Patenterteilung:
**01.12.2010 Patentblatt 2010/48**

(21) Anmeldenummer: **08716096.6**

(22) Anmeldetag: **28.02.2008**

(51) Int Cl.:
*A61B 5/00* *(2006.01)*     *G01N 21/64* *(2006.01)*

(86) Internationale Anmeldenummer:
**PCT/EP2008/001567**

(87) Internationale Veröffentlichungsnummer:
**WO 2008/113461 (25.09.2008 Gazette 2008/39)**

(54) **VERFAHREN UND VORRICHTUNG ZUM AUSWERTEN VON FLUORESZENZBILDSÄTZEN UND VORRICHTUNG ZU SEINER DURCHFÜHRUNG**

METHOD AND DEVICE FOR EVALUATING FLUORESCENCE IMAGE RECORDS

PROCÉDÉ ET DISPOSITIF D'ÉVALUATION D'ENSEMBLES D'IMAGES DE FLUORESCENCE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.03.2007 DE 102007014413**

(43) Veröffentlichungstag der Anmeldung:
**25.11.2009 Patentblatt 2009/48**

(73) Patentinhaber: **Dürr Dental AG**
**74321 Bietigheim-Bissingen (DE)**

(72) Erfinder:
• **THOMS, Michael**
**74321 Bietigheim-Bissingen (DE)**
• **MAIER Raimund**
**71732 Tamm (DE)**

(74) Vertreter: **Ostertag, Reinhard et al**
**Patentanwälte**
**Ostertag & Partner**
**Epplestr. 14**
**70597 Stuttgart (DE)**

(56) Entgegenhaltungen:
**DE-A1- 10 207 918    DE-A1-102004 024 494**

**Beschreibung**

Die Erfindung betrifft ein Verfahren zum Auswerten von Fluoreszenzbildsätzen und eine Vorrichtung zu seiner Durchführung.

Es ist z.B. aus der DE 10 2004 491 A1 bekannt, dass sich kranke und gesunde Gewebe durch ihre Farbe unterscheiden. Bestrahlt man Zahnschmelz oder ähnliches Gewebe mit blauem oder ultraviolettem Licht, so fluoresziert das Gewebe, wobei man bei gesunden und kranken Geweben unterschiedliche spektrale Anteile im Fluoreszenzlicht beobachtet, z.b. unterschiedliche Rot- und Grünanteile.

Man kann nun die Änderung des Rotanteils oder die Änderung des Grünanteils dazu verwenden, krankes Gewebe zu erkennen. Ein besonders empfindlicher Nachweis kranken Gewebes gelingt dann, wenn man das Verhältnis zwischen Rotanteil und Grünanteil des Fluoreszenzlichts untersucht (DE 10 2004 024 494 A1).

Dokument DE 10 207 918 A1 offenbart ein Verfahren gemäss der Präambel des Anspruchs 1.

Bei den bekannten Verfahren zum Erkennen kranker Gewebe aus Fluoreszensbildern ist noch nachteilig, dass das Ergebnis durch Nebenlicht (Störlicht) beeinflusst wird.

Durch die vorliegende Erfindung soll daher ein Verfahren und eine Vorrichtung zum Auswerten von von der gleichen Aufnahmestelle gemachten Fluoreszenzbildern angegeben werden, bei welchem die Störeinflüsse von Neben- oder Störlicht vermindert sind.

Diese Aufgabe ist erfindungsgemäß gelöst durch ein Verfahren gemäß Anspruch 1 sowie durch eine Vorrichtung zu seiner Durchführung gemäß Anspruch 17.

Erfindungsgemäß wird aus zwei bei unterschiedlichen Wellenlängen aufgenommenen Teilbildern der gleichen Stelle eines Objektes (Gewebe) ein Quotientenbild erstellt, welches pixelweise das Verhältnis der Intensitäten im ersten Teilbild und im zweiten Teilbild wiedergibt. Für dieses Quotientenbild wird ein Verteilungsspektrum berechnet, das angibt, mit welcher Häufigkeit welcher Quotient zwischen Teilbildsignalen erhalten wird. Dieses Spektrum ist durch eine Verteilungskurve charakterisiert, welche ein Maximum hat und durch eine Breite z. B. eine Halbwertsbreite charakterisiert ist.

Die Form dieser Verteilungskurve ist weitestgehend unabhängig von Störlichteinflüssen. Durch aus der Umgebung stammendes Störlicht wird die Verteilungskurve nur je nach Farbe des Störlichts mehr oder weniger weit in Abszissenrichtung des Koordinatensystems, das zur Darstellng der Verteilungskurve verwendet wird, verschoben und in einigen Fällen auch verbreitert.

Erfindungsgemäß werden nun die störlichtunabhängigen charakteristischen Eigenschaften der Verteilungskurve dazu verwendet, einen Schwellwert zu bestimmen, der bei der Unterscheidung zwischen krankem und gesundem Gewebe verwendet wird. Es wird unter Verwendung dieses Schwellwertes ein Kontrastbild erstellt, bei welchem z.B. diejenigen Pixel verwendet werden, für welche der wie vorstehend beschrieben berechnete Schwellwert überschritten ist.

Auf diese Weise erhält man eine sehr aussagekräftige Darstellung kranker Gewebebereiche im Kontrastbild und kann auf diesem kranke Gewebebereiche sicher ausmachen, die auf dem ursprünglichen Gesamtbild des Objekts nicht deutlich zu sehen waren.

Vorteilhafte Weiterbildungen der Erfindung sind in Unteransprüchen angegeben.

Ein gemäß Anspruch 2 erzeugtes Kontrastbild zeichnet sich durch besonders augenfällige Unterscheidung von krankem und nicht krankem Gewebe aus.

Bildet man das Quotientenbild direkt aus den Intensitäten der Pixel des Gesamtbilds, so ist das Quotientenbild insbesondere in den Bereichen kleiner Intensität stark verrauscht, da kleine Änderungen im Nenner eines Bruchs zu großen Änderungen im Wert des Bruchs führen. Bei einem Verfahren gemäß Anspruch 3 ist dieses durch die Quotientenbildung hervorgerufene Rauschen geschwächt, wobei die Schwächung umso stärker ist, je größer die zu den Teilbildsignalen hinzuaddierte Konstante ist. Mit wachsender Konstante wird umgekehrt natürlich die Empfindlichkeit der Diagnose herabgesetzt. Durch geeignete Wahl der Konstante, für die man aus früheren Aufnahmen einen Ausgangswert vorgeben kann, der dann im Einzelfall an die aktuellen Bedingungen der Gewebeaufnahme angepasst werden können, kann man einen für die Praxis brauchbaren Kompromiss zwischen Rauschfreiheit und Empfindlichkeit der Diagnose erzielen.

Das Verfahren nach Anspruch 4 trägt stark unterschiedlichen Verstärkungsfaktoren für die Pixelintensitäten der Teilbilder Rechnung, wenn solche beim Farbabgleich verwendet werden.

Mit der Weiterbildung der Erfindung gemäß Anspruch 5 wird erreicht, dass durch das Entrauschen der Quotientenbildung der Dynamikbereich des Quotientenbildes dem des Gesamtbildes nahekommt.

Gemäß Anspruch 6 kann man die Glättung des Quotientenbilds gemäß dem jeweils angefundenen Rauschanteil in den Teilbildern einstellen.

Der Anspruch 7 gibt bevorzugte Werte für die Konstante an, die beim Erzeugen eines nicht verrauschten Quotientenbilds verwendbar sind.

Oft lässt sich die aus dem Quotientenbild abgeleitete Verteilungskurve durch eine analytische Kurve darstellen, und in diesem Fall kann man dann gemäß Anspruch 8 auf rechnerische Weise automatisch einfach die interessierenden Kenngrößen der Verteilungskurve (Maximum und Breite) bestimmen.

Für viele Anwendungsfälle ist eine verteilungskurve in Form einer Gauß-Kurve oder Normalverteilung besonders günstig, wie im Anspruch 9 angegeben ist.

Gemäß Anspruch 10 kann man den Schwellwert, der für die Unterscheidung zwischen krankem und gesundem Gewebe verwendet wird, angepasst an die jeweils angefundenen Aufnahmebedingungen vorgeben.

Dabei werden bei dem Verfahren nach Anspruch 11 solche Teile der Verteilungskurve nicht berücksichtigt, die mit stärkeren systembedingten oder zufälligen Unsicherheiten behaftet sind.

Die Trennung zwischen verwerteten und nicht berücksichtigten Abschnitten der Verteilungskurve lässt sich gemäß Anspruch 12 auf sehr einfache Weise und rasch bewerkstelligen.

Dabei haben sich die im Anspruch 13 angegebenen Werte als besonders geeignet erwiesen, Fußabschnitte der Verteilungskurve, die von Zufälligkeiten stark betroffen sind, abzuschneiden.

Mit dem im Anspruch 14 angegebenen Verfahren wird eine nochmls bessere automatische Anpassung des zwischen krankem und gesundem Wert unterscheidenden Schwellwerts an die aktuelle Aufnahmesituation erhalten. Dabei ist einerseits die Gefahr ausgeräumt, dass kranke Bereiche nicht erkannt werden, andererseits die Gefahr klein gehalten, dass gesunde Bereiche fälschlicherweise als kranke dargestellt werden.

Die im Anspruch 15 angegebene Gewichtung zwischen den beiden Teilfaktoren, welche den Schwellwert bestimmen, hat sich als besonders günstig herausgestellt.

Die Weiterbildung der Erfindung gemäß Anspruch 16 gestattet es, kranke Bereiche und das Gesamtbild des Objekts leicht erfassbar zueinander in Relation zu bringen. Man kann leicht sehen, welche Bereiche des Objekts krankhaft verändert sind.

Eine Vorrichtung, wie sie im Anspruch 18 angegeben ist, lässt sich sehr einfach unter Verwendung handelsüblicher und preisgünstiger Komponenten bauen.

Eine Vorrichtung gemäß Anspruch 19 ist wieder im Hinblick auf die räumliche Zuordnung von kranken Gewebebereichen und Gesamtobjekt von Vorteil.

Bei einer Vorrichtung gemäß Anspruch 20 kann man verschiedene interessierende Bilder vom Objekt wahlweise abrufen.

Nachstehend wird die Erfindung anhand eines Ausführungsbeispiels unter Bezugnahme auf die Zeichnung näher erläutert. In dieser zeigen:

Figur 1: Eine schematische Darstellung einer dentalen Fluoreszenzkamera sowie ein Blockschaltbild einer mit dieser verwendeten Betriebs- und Aus- werteeinheit;

Figur 2: Ein Gesamtbild der Kaufläche eines Zahns mit einer Initialläsion/Fissur;

Figur 3: Drei Teilbilder der in Figur 2 gezeigten Kau- fläche, welche den Farben rot, grün und blau entsprechen;

Figur 4: Ein Quotientenbild, welches durch pixelweise Division des Rotbilds durch das Grünbild der Kaufläche erhalten wurde;

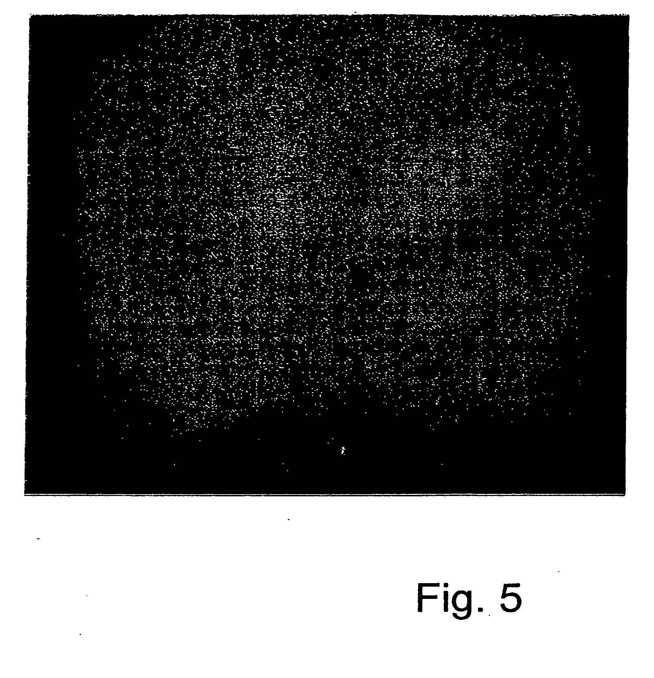

Figur 5: Ein modifiziertes Gesamtbild der Kaufläche, welches durch Multiplikation des Gesamtbilds von Figur 2 mit dem Quotientenbild von Figur 4 erhalten wurde;

Figur 6: Die Häufigkeitsverteilung von Pixeln im Quotien- tenbild aufgetragen über dem Rot/Grünverhältnis der Pixel-Bildsignale; und

Figur 7: Ein Kontrastbild, welches unter Berücksichtigung der Pixelverteilung von Figur 6 aus dem Quotien- tenbild von Figur 4 abgeleitet wurde.

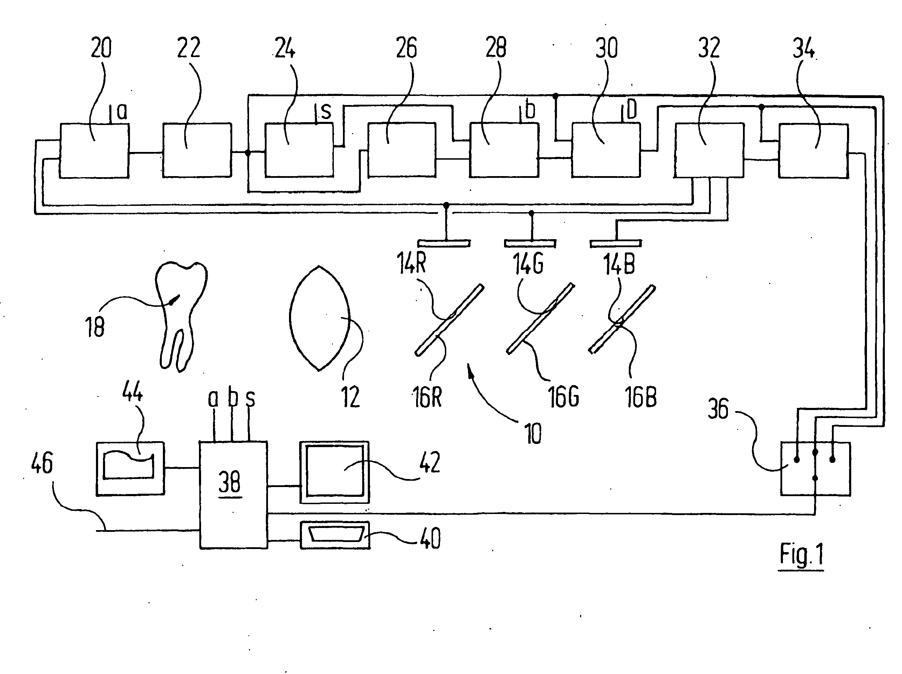

In Figur 1 ist mit 10 insgesamt ein dentales Fluoreszenz-Kamerahandstück bezeichnet. Es umfasst eine Optik 12, die schematisch als einzelne Linse dargestellt ist, drei Bildwandler 14R, 14G und 14B für die Farben rot, grün und blau, die über die dichroitische Strahlteiler 16R, 16G und 16B mit dem durch die Optik 12 von einem Zahn 18 erzeugten Bild beaufschlagt sind.

Soweit nachstehend Ausführungen gemacht werden, die für alle Bildwandler bzw. alle Filter gleichermaßen gelten, wird auf den Zusatz der Buchstaben R, G und B verzichtet.

Die Bildwandler 14 sind in Figur 1 aus Gründen der übersichtlichen Darstellung gleich weit von der optischen Achse des Kamerahandstücks 10 entfernt dargestellt. In Wirklichkeit sind die Abstände unterschiedlich, derart, dass der gesamte Lichtweg zwischen Optik 12 und Bildwandler für die verschiedenen Bildwandler gleich groß ist.

Die Bildwandler 14 können übliche CCD- oder CMOS-Bildwandler sein und speichern jeweils ein Bild, welches in Zeilen und Spalten angeordnete Pixel umfasst. Das Auslesen dieser Pixel erfolgt zeilen- oder spaltenweise, wie von derartigen Bildwandlern her bekannt.

Für die Zwecke der vorliegenden Beschreibung sei angenommen, dass die Bildwandler und verschiedene mit ihnen verbundene später genauer zu beschreibende Schaltkreise gegebenenfalls jeweils einen Speicher aufweisen, der ein gesamtes Bild halten kann. Diese Speicher sind an sich bekannt und brauchen im Einzelnen nicht beschrieben zu werden. Gegebenenfalls hat jeder der Schaltkreise einen eingangsseitigen und einen ausgangsseitigen Speicher, so dass der Schaltkreis selbst und mit ihm verbundene Schaltkreise jeweils auf ein volles Bild zugreifen können.

Die Bildwandler 14R und 14G sind mit zwei Eingängen eines Rechenkreises 20 verbunden, der aus den entsprechenden Teilbildern im Roten und Grünen ein Quotientenbild erzeugt. Dies erfolgt pixelweise, indem für jedes Pixel i mit der Intensität $I_R(i)$ bzw. $I_G(i)$ den Signalquotienten $Q_{RG}(1)$ berechnet wird. Dabei wird vor der Quotientenbildung jedem der Signale $I_R(i)$ und $I_G(i)$ eine Konstante $a_R$ bzw. $a_G$ hinzugezählt, welche der Rechenkreis 20 von außen erhält.

Die Pixel Q(i) des Quotientenbildes sind somit gegeben durch die Gleichung

$$Q(i) = (I_R(i) + a_R) / (I_G(i) + a_G),$$

wobei man vorzugsweise die Bedingung

$$a_R / a_G = \text{Mittelwert} (I_R(i) / I_G(I))$$

einhält.

Unterscheiden sich die gemittelten Intensitäten nicht erheblich, kann man näherungsweise

$$a_R = a_G = a$$

wählen.

Durch Hinzufügen dieser Konstanten $a_R$ und $a_G$ bzw. a wird erreicht, dass sich bei kleinen Signalintensitäten $I_G(i)$ durch Rauschen bedingte Schwankungen nicht in großen Änderungen des Quotienten niederschlägt.

In der Praxis hat sich ein Wert für a im Bereich zwischen 30 und 120, vorzugsweise zwischen 50 und 100 nochmal vorzugsweise von etwa 80 bewährt.

Für sehr kleine Intensitäten $I_R$ und $I_G$ erhält man bei Verwendung eines einzigen a für das Intensitätsverhältnis näherungsweise den Wert 1, der auch für gesundes Gewebe durch Einstellung der Verstärkungsfaktoren für $I_R$ und $I_R$ beim Farbabgleich erhalten wird. Ist der Farbabgleich für $I_R$ und $I_G$ durch sehr unterschiedliche Verstärkungsfaktoren gegeben, ist es zweckmäßig mit unterschiedlichen $a_R$ und $a_G$ zu arbeiten, wie oben angesprochen.

Am Ausgang des Rechenkreises 20 wird somit ein Quotientenbild erhalten, dessen Pixel jeweils zeigen, wie groß das Verhältnis zwischen Rotanteil und Grünanteil im ursprünglichen Gesamtbild des Zahns 18 ist.

Ein weiterer Rechenkreis 22 ist eingangsseitig mit dem Quotientenbild beaufschlagt und berechnet aus diesem eine Verteilungskurve des Quotientenbilds, d. h. er stellt fest, wie groß die Häufigkeit H von Pixeln Q(i) ist, für welche der Quotient Q(i) einen bestimmten Wert q hat.

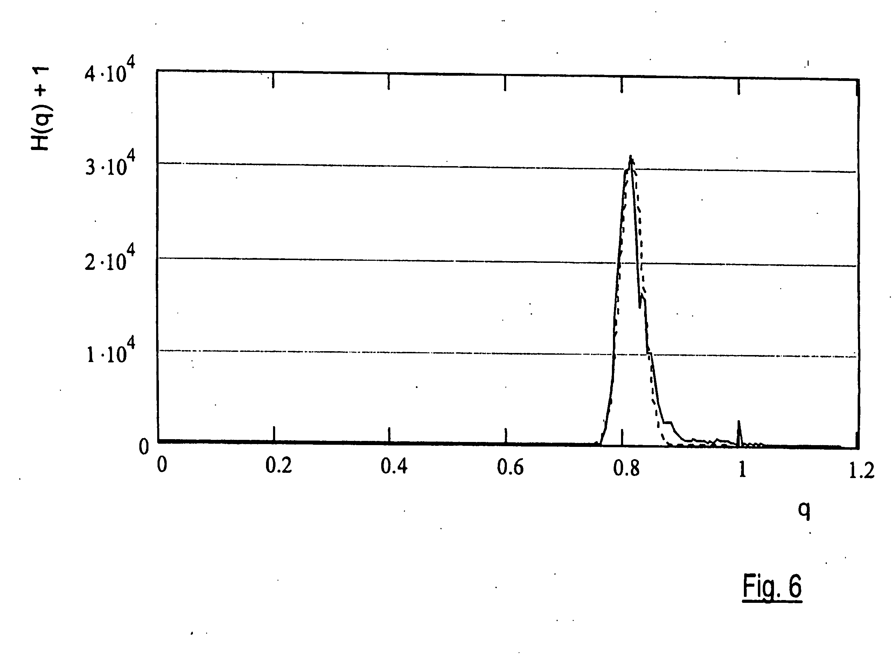

Das Ergebnis, welcher der Rechenkreis 22 bereitstellt, ist eine Verteilungskurve, wie sie in Figur 6 dargestellt ist.

Mit dieser Verteilungskurve ist ein weiterer Rechenkreis 24 beaufschlagt, der für die Verteilungskurve die Breite bestimmt.

Der Rechenkreis 24 kann so arbeiten, dass er einfach aus der gemessenen Verteilungskurve die Halbwertsbreite bestimmt. Er kann aber auch vorzugsweise so arbeiten, dass man an die gemessene Verteilungskurve eine analytische Kurve durch Least Square Fit anpasst. Dabei wird als Verteilungsfunktion vorzugsweise eine Gauß-Funktion gewählt.

Diese hat dann im vorliegenden Fall die Form

$$H(q) = A \exp (-0,5(q-\mu)/\sigma)^2,$$

wobei $\mu$ der Q-Wert ist, bei welchem das Maximum das Gaußkurve liegt, und $\sigma$ die Standardabweichung der Gaußkurve ist.

Im Einzelnen arbeitet der Rechenkreis 24 so, dass er von den gemessenen Quotientensignalen nur diejenigen verwendet, deren Wert größer ist als eine vorgegebene Schwelle s, die dem Rechenkreis von außen mitgeteilt wird. Auf diese Weise ist gewährleistet, dass sich Zufälligkeiten in dem Quotienten intensitätsarmer Pixel nicht in die Bestimmung der Amplitude und der Halbwertsbreite der Gauß-Funktion fortpflanzen.

Das Ausgangssignal des Rechenkreises 24 wird auch auf einen weiteren Rechenkreis 26 gegeben, der den Mittelwert m der Verteilungskurve berechnet. Dies kann z. B. analytisch unter Zugrundelegung von Amplitude und Standardabweichung der ermittelten Gauß-Funktion erfolgen.

Ein weiterer Rechenkreis 28 dient dazu, einen Schwellwert S zu berechnen, der die Unterscheidung zwischen krankem und gesundem Gewebe ermöglichen soll. Dieser Schwellwert wird nach der Gleichung

$$S = m + b * \sigma$$

berechnet. Dabei ist m der wie oben beschrieben berechnete Mittelwert der Verteilungskurve und σ deren Standardabweichung.

Ein weiterer Rechenkreis 30 ist zum Einen mit dem Schwellwert S, zum Anderen mit dem Quotientenbild beaufschlagt. Er modifiziert das Quotientenbild pixelweise unter Berücksichtigung der Intensität des einzelnen Pixels und des Schwellwerts S.

Die Modifizierung kann eine Intensitätsänderung und/oder eine Farbänderung sein. Vorzugsweise wird immer auch eine Intensitätsänderung vorgenommen.

Für diejenigen Pixel, deren Intensität größer ist als der Schwellwert, wird z.B. die Intensität nochmals angehoben, für diejenigen Pixel, für die die Pixelintensität kleiner ist als der Schwellwert, wird die Intensität abgeschwächt. Auf diese Weise erhält man ein modifiziertes Quotientenbild oder Kontrastbild, in welchem die kranken Gewebebereiche stark vorgehoben sind.

Diese kranken Gewebebereiche erscheinen somit über einem schwachen Bild der gesunden Bereiche des Zahns 18.

In Abwandlung kann man die Konstrastverschärfung auch extrem so vornehmen, dass man den Pixeln mit Intensitäten, die größer sind als der Schwellwert S eine volle Maximale Helligkeit gibt, während man den anderen Pixeln die Helligkeit 0 zuordnet. Man erhält so ein sehr konstrastreiches Bild der kranken Gewebebereiche.

Wie aus Figur 1 ersichtlich, sind die drei Wandler 14R, 14G, 14B zusätzlich mit den Eingängen eines Überlagerungskreises 32 verbunden, an dessen Ausgang somit ein Gesamtbild bereitgestellt wird, das den beobachteten Zahnbereich unverfälscht wiedergibt.

In einem nachgeschalteten Mischkreis 34 kann man das unverfälschte Gesamtbild des Zahns mit den im Rechenkreis 30 berechneten Kontrastbild verketten z. B. durch pixelweise Multiplikation der Intensitäten der zu verkettenden Bilder.

Über einen Umschalter 36 kann man die verschiedenen oben angesprochenen Bilder und die Verteilungskurve der Pixel des Quotientenbilds über das Rot/Grünverhältnis wahlweise an einen Rechner 38 überstellen, der das gesamte Gerät steuert. Dieser Rechner stellt die Konstanten a, b und S bereit, die in den Rechenkreisen 20, 22 und 28 benötigt werden. Die Bereitstellung dieser Konstanten erfolgt ausgehend von Grundwerten, die in einem mit dem Rechner 38 verbundenen Massenspeicher abgelegt sind unter Modifikation gemäß dem Einzelfall Rechnung tragender Korrekturen, die an einem mit dem Rechner Tastenfeld 40 eingegeben werden.

An einem mit dem Rechner 38 verbundenen Monitor 42 können die verschiedenen oben angesprochenen Bilder dargestellt werden. Diese Bilder können auch über einen Drucker 44 bleibend dokumentiert werden.

Zur Übertragung der verschiedenen Bilder an ein zentrales Archiv und zum Austauschen von Patientendaten mit einem zentralen Archiv ist der Rechner 38 an eine Datenleitung 46 angeschlossen, die Teil eines Netzwerks sein kann.

Mit dem oben beschriebenen Diagnosegerät können insbesondere mit Bakterien belegte Bereiche von Geweben erkannt werden, z. B. auf Zahnoberflächen.

Bestrahlt man den Zahn 18 mit einer UV-Lichtquelle UV mit ultraviolettem Licht, so hat man in gesunden Bereichen eine Fluoreszenz, die im Grünen stark ist. In Oberflächenbereichen des Zahns, die mit Bakterien belegt sind, ist dagegen die Fluoreszenz im Roten stärker.

Mit zunehmendem Angriff der Bakterien auf das gesunde Zahnmaterial wird das grüne Fluoreszenzbild reduziert. Durch Beobachtung des Verhältnisses zwischen rotem und grünem Fluoreszenzbild kann man sich somit einen Überblick über gesunde und kranke Zahnbereiche verschaffen.

Wie oben dargelegt, lässt sich der Kontrast zwischen gesundem und krankem Zahngewebe dadurch verbessern, dass man pixelweise den. Quotienten zwischen rotem Teilbild des Zahns und grünem Teilbild des Zahns bildet, wobei man den roten und grünen Teilsignalen jeweils die Konstante a vor der Quotientenbildung hinzuaddiert, um eine stärkere Verrauschung des Quotientensignals zu vermeiden.

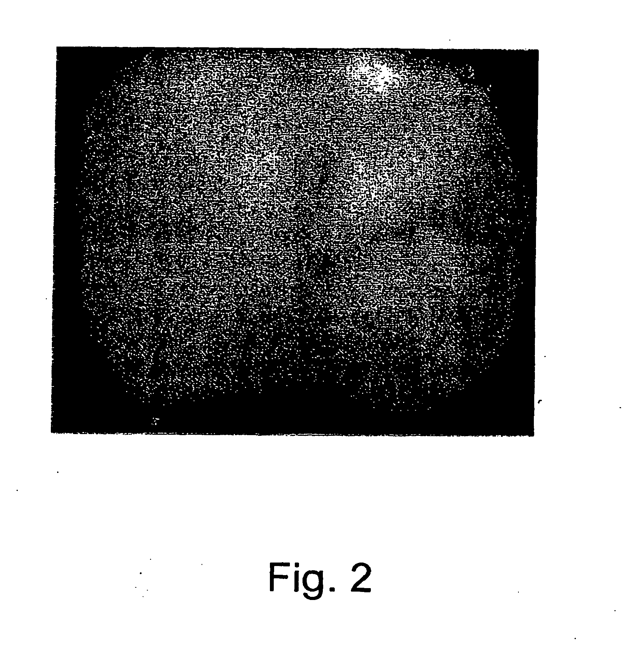

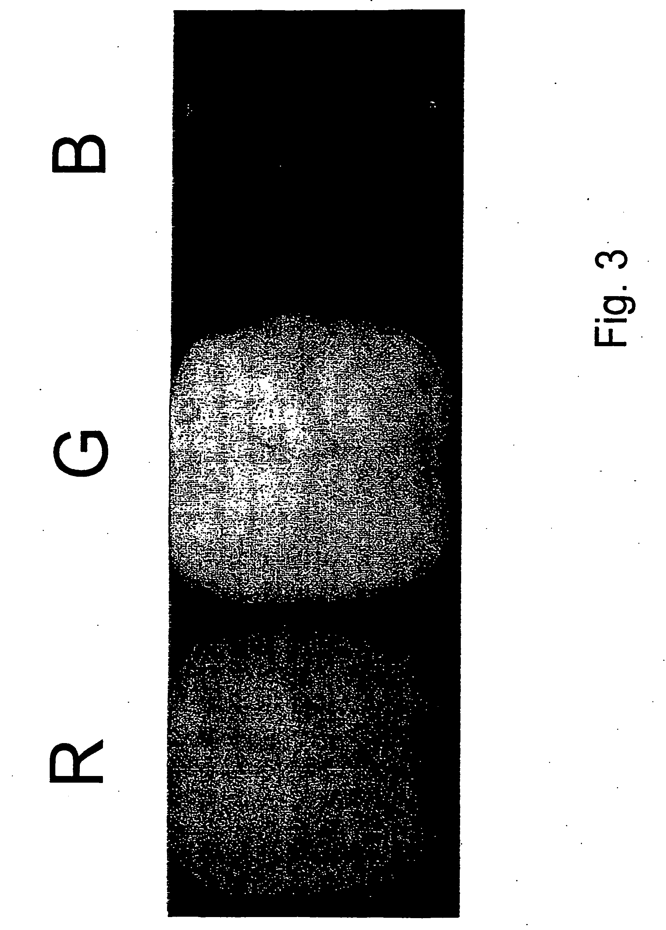

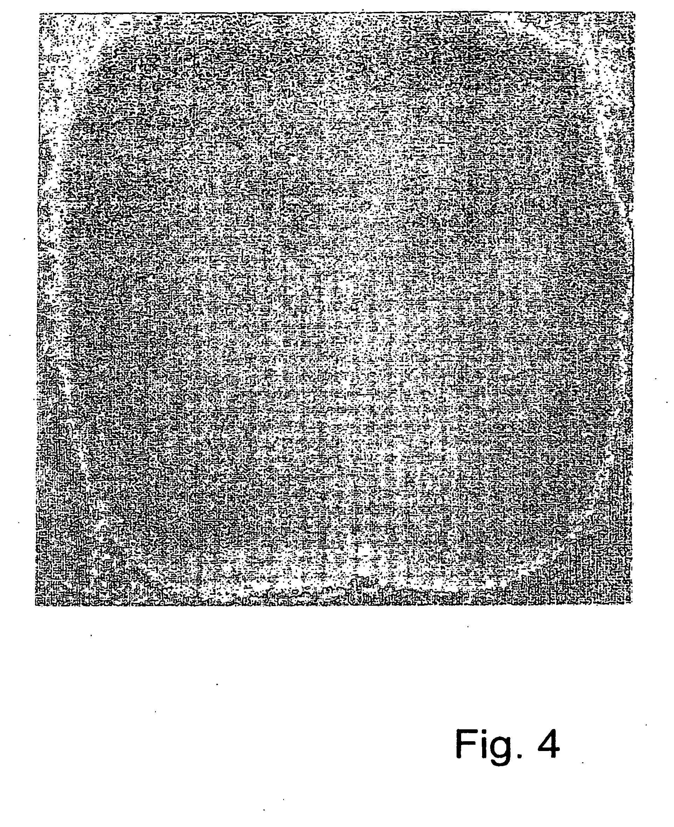

Aus den Figuren 2 bis 4 ist ersichtlich, dass durch Erzeugen des Quotientensignals schon eine bessere Hervorhebung einer Initialläsur erfolgt, die im Bereich der Fissur des Zahns vorliegt.

Figur 6 zeigt die Häufigkeitsverteilung H(q) der Pixelintensitäten des Quotientenbilds.

Beim Erstellen dieser Häufigkeitsverteilung wurden Werte mit Intensität 0 im roten oder grünen Kanal nicht berücksichtigt.

In der Verteilungskurve der Helligkeiten des Quotientenbilds nach Figur 6 liegt das Maximum der verteilungskurve bei 0,813. Nach dem oben Gesagten, liegen erkrankte Bereiche bei höheren Werten des Rot/Grün-Quotienten q.

In Figur 6 ist gestrichelt eine durch Least Square Fit erhaltene Gauß-Verteilung dargestellt. Die Anpassung der Gaußkurve erfolgte nur für diejenigen Messpunkte, bei denen die Häufigkeit H nicht weniger als 15 % der maximalen Häufigkeit Hmax beträgt.

Für diese Kurve errechnet sich eine Standard-Abweichung $\sigma$ von 0,0194 und ein Mittelwert der Verteilung über einen Bereich um das Maximum herum von m = 0,813 . Wie gesagt, werden für diese beiden Berechnungen nur Punkte der Verteilungskurve berücksichtigt, bei denen die Häufigkeit H (q) mehr als 15 % des Maximums beträgt.

Die Schwelle S für den Rechenkreis 30 wird gemäß der Formel

$$S = m + b\sigma$$

berechnet, wobei $b \approx 3,4$ gewählt wird.

Dies hat zur Folge, dass in einem Bild mit $10^6$ Bildpunkten bei einem statistischen Rauschen des Bildsignals nur etwa ein Anteil von $1,2 \times 10^{-6}$ der Bildpunkte bei dieser Schwelle liegen und noch weniger oberhalb der Schwelle. Dies bedeutet in der Praxis, dass es praktisch nicht vorkommt, dass gesunde Bildpunkte fälschlicherweise als kranke gemeldet werden.

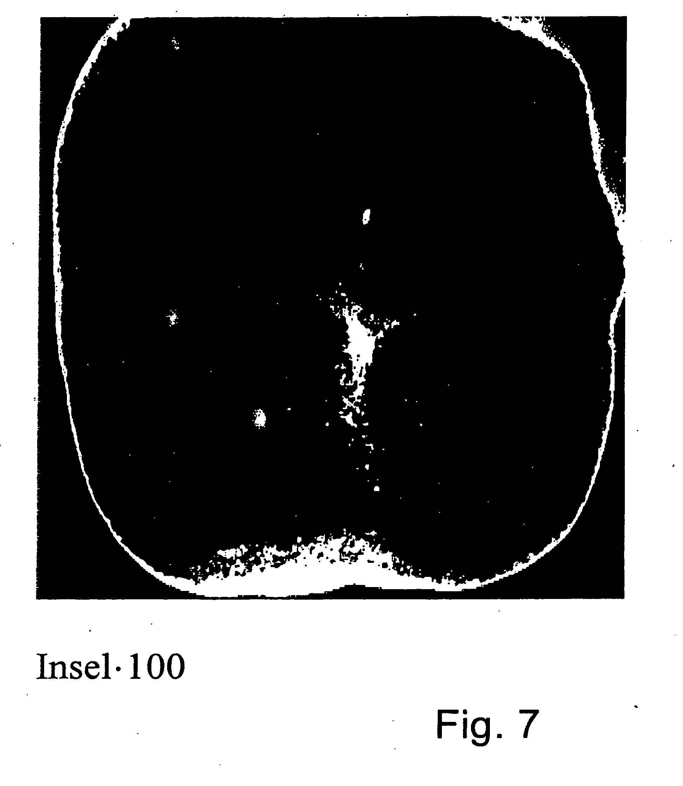

In Figur 7 hat man die oben ansprochene scharfe Kontrastvergrößerung des Kontrastbilds vorgenommen, bei welcher kranke Bereiche die volle Helligkeit erhalten und gesunde Bereiche eine vorgegebene kleine Helligkeit erhalten. Man erkennt, dass eine im Gesamtbild von Figur 2 noch unscheinbare Stelle im Kontrastbild nach Figur 7 als kranke Stelle erscheint. An dieser Stelle wurden bei gezielter Suche anhand des Kontrastbilds später durch Einfärben auch in der Tat Bakterien gefunden.

Der Vorteil des oben beschriebenen Verfahrens und der oben beschriebenen Vorrichtung liegt insbesondere darin, dass unabhängig von einer Verschiebung des Maximums der verteilungskurve nach Figur 6, wie sie durch Fremdlicht bedingt sein kann, immer das gleiche Ergebnis erzielt wird.

Da das oben geschilderte Verfahren mit einer statistischen Auswertung des Quotientenbilds arbeitet, wird verhindert, dass bei stark verrauschten Signalen gesunde Gewebebereiche fälschlicherweise als krank eingestuft werden.

In der Zahnheilkunde werden die Läsionen des Zahnhartgewebes mit Kennzeichnung D0, D1, D2 und D3 versehen. Mit dem oben beschriebenen Verfahren und dem oben beschriebenen Gerät lassen sich Kontrastbilder erstellen, die gezielt Läsionen ab einer bestimmten Schwere darstellen. Dies lässt sich durch entsprechende Wahl der Schwellwerte S auf einfache Weise realisieren.

Durch das geschilderte Vorgehen ist ebenfalls vermieden, dass Nebenlicht die Ermittlung der erkrankten Bereiche verfälscht.

Obenstehend wurde die Erfindung unter Bezugnahme auf die Erkennung kranken Zahnhartgewebes beschrieben. Es versteht sich, dass man das Verfahren und die Vorrichtung auch bei anderen erkrankten Geweben verwenden kann, z. B. bei Haut- oder Hirntumoren, bei welchen der Rand des erkrankten Bereichs ermittelt werden soll. Voraussetzung ist nur, dass sich gesunde Bereiche durch ein bestimmtes nur wenig örtlich schwankendes Intensitätsverhältnis von rotem und grünem Teilbild charakterisieren lassen.

Es wurde oben ein Verfahren und eine Vorrichtung zum Auswerten von von der gleichen Stelle eines Objektes bei unterschiedlichen Wellenlängen aufgenommenen Fluoreszenzbildern (Bildersatz) beschrieben, welche unabhängig von Störlicht arbeiten. Mit einer Kamera werden zwei Teilbilder des Gewebes im Roten und Grünen erzeugt. Aus diesen beiden Teilbildern wird ein Quotientenbild pixelweise erzeugt, und für dieses Quotientenbild wird bestimmt, mit welcher Häufigkeit Bildpunkte mit gleichem Farbverhältnis auftreten, die konkret also ein vorgegebenes Rot/Grünverhältnis haben. Für die so erhaltene Verteilungskurve werden Mittelwert und Breite bestimmt. Diese beiden Endgrößen der Verteilungskurve werden dazu verwendet, einen Schwellwert zu berechnen, der zur Vorgabe einer Kontrastschärfung verwendet wird. Unter Verwendung dieses Schwellwerts wird dann das Quotientenbild so modifiziert, dass z.B. kranken Gewebebereichen entsprechende Bildpartien stark hervorgehoben werden.

Das Verfahren und die Vorrichtung lassen sich auch verwenden, wenn eine Abschirmung von Neben- oder Störlicht nicht möglich ist.

**Patentansprüche**

1. Verfahren zum Auswerten von Fluoreszenz-Teilbildern unterschiedlicher Wellenlänge, die unter Einstrahlung von Anregungslicht von der selben Stelle eines Objektes gewonnen sind, wobei aus den beiden Teilbildern ein Quotientenbild durch pixelweises Dividieren der Pixelintensitäten der beiden Teilbilder erzeugt wird und für das Quotientenbild eine Verteilungskurve bestimmt wird, die angibt, mit welcher Häufigkeit ein bestimmter Quotient auftritt, **darduch gekennzeichnet, dass** in Abhängigkeit von der Breite und/oder dem Mittelwert der Verteilungskurve ein Schwellwert bestimmt wird und dass ein Diagnosebild aus dem Quotientenbild erzeugt wird, bei welchem die Pixel des Quotientenbilds unterschiedlich modifiziert werden, je nachdem, wie ihre Intensität zum Schwellwert liegt, wodurch ein Kontrastbild erhalten wird.

2. Verfahren nach Anspruch 1, **dadurch gekennzeichnet, dass** Pixel des Quotientenbildes in der Intensität verstärkt werden, insbesondere auf maximale Intensität werden, und/oder eine erste Farbe gesetzt werden, wenn ihre Intensität größer als der Schwellwert ist, und in der Intensität geschwächt werden, insbesondere auf minimale Intensität gesetzt werden, und/oder auf eine zweite Farbe gesetzt werden, wenn ihre Intensität kleiner als der Schwellwert ist.

3. Verfahren nach Anspruch 1 oder 2, **dadurch gekennzeichnet, dass** zu den Pixelsignalen der beiden Teilbilder vor der Quotientenbildung jeweils eine Konstante hinzugezählt wird.

4. Verfahren nach Anspruch 3, **dadurch gekennzeichnet, dass** das Verhältnis der beiden Konstanten gleich dem Verhältnis der Mittelwerte der Pixelintensitäten der beiden Teibilder ist.

5. Verfahren nach Anspruch 4, **dadurch gekennzeichnet, dass** den Pixelsignalen beider Teilbilder die gleiche Konstante hinzugefügt wird.

6. Verfahren nach Anspruch 3, 4 oder 5, **dadurch gekennzeichnet, dass** die den Pixelsignalen der Teilbilder hinzugefügte Konstante in Abhängigkeit vom Signal/ RauschVerhältnis der Teilbilder gewählt ist.

7. Verfahren nach Anspruch 6, **dadurch gekennzeichnet, dass** bei Anregung der Fluoreszenz mit blauem Licht oder UV-Licht das erste Teilbild im Roten und das zweite Teilbild im Grünen erzeugt wird und die hinzuzufügende Konstante zwischen 30 und 120, vorzugsweise zwischen 50 , und 100, nochmal vorzugsweise etwa 80 beträgt.

8. Verfahren nach einem der Ansprüche 1 bis 7, **dadurch gekennzeichnet, dass** die Breite der Verteilungskurve des Quotientenbilds derart bestimmt wird, dass eine analytische Verteilungsfunktion an die Verteilungskurve angepasst wird, insbesondere durch einen Least Square Fit, und die Breite der Verteilungskurve und/oder deren Maximum und/oder deren Mittelwert durch die entsprechenden Werte der analytischen Kurve vorgegeben werden.

9. Verfahren nach Anspruch 8, **dadurch gekennzeichnet, dass** die analytische Verteilungsfunktion eine Gauss-Funktion ist.

10. Verfahren nach einem der Ansprüche 1 bis 9, **dadurch gekennzeichnet, dass** der Schwellwert in Abhängigkeit vom Mittelwert der Verteilungskurve berechnet wird.

11. Verfahren nach Anspruch 10, **dadurch gekennzeichnet, dass** man Bereiche der Verteilungskurve unberücksichtigt lässt, für welche die Häufigkeit einen vorgegebenen Mindestwert unterschreitet.

12. Verfahren nach Anspruch 11, **dadurch gekennzeichnet, dass** der Mittelwert der Verteilungskurve unter Berücksichtigung derjenigen Kurvenbereiche berechnet wird, in denen die Amplitude größer ist als ein vorgegebener Bruchteil des Maximums der verteilungskurve.

13. Verfahren nach Anspruch 12, **dadurch gekennzeichnet, dass** der vorgegebene Bruchteil zwischen 0,05 und 0,3 beträgt, vorzugsweise zwischen 0,1 und 0,2, nochmal vorzugsweise etwa 0,15.

14. Verfahren nach einem der Ansprüche 10 bis 13, **dadurch gekennzeichnet, dass** der Schwellwert aus dem Mittelwert der Verteilungskurve und der mit einer Konstanten multiplizierten Breite der Verteilungskurve bestimmt wird.

15. Verfahren nach Anspruch 14, **dadurch gekennzeichnet, dass** die Konstante zwischen 2 und 5, vorzugsweise etwa 3,5 beträgt.

16. Verfahren nach einem der Ansprüche 1 bis 15, **dadurch gekennzeichnet, dass** ein nicht gefiltertes Gesamtbild oder ein Teilbild des Objektes mit dem Kontrastbild verkettet, insbesondere multipliziert wird.

17. Vorrichtung zur Durchführung des Verfahrens nach einem der Ansprüche 1 bis 16, mit einer Kamera (10), welche von einem Objekt (18) mindestens zwei Teilbilder unterschiedlicher Farbe bereitstellt, mit einem Rechenkreis (20), welcher aus den beiden Teilbildern ein Quotientenbild erzeugt, indem er pixelweise die Pixelsignale des ersten Teilbilds durch diejenigen des zweiten Teilbilds dividiert, und mit einem Analysator (24), welcher eine Verteilungskurve des Quotientenbildes erzeugt, welche die Häufigkeit des Auftretens von Pixeln mit einem vorgegebenen Farbenverhältnis im Quotientbild darstellt, **dadurch gekennzeichnet, dass** der Analysator (24) ferner das Maxi- um und/oder die Breite dieser Verteilungskurve bestimmt, und dass ein Kontrastbild-Rechenkreis (30) unter Verwendung zumindest derjenigen Pixel des Quotientenbilds, die einen vorgegebenen Schwellwert überschreitet, die Pixel eines Diagnosebilds bildet.

18. Vorrichtung nach Anspruch 17, **dadurch gekennzeichnet, dass** die Kamera (10) einen CCD- oder CMOS-Bildwandler (14R, 14G, 14B) zur Erzeugung von RGB-Teibildern für die drei Farben rot, grün und blau umfasst.

19. Vorrichtung nach Anspruch 17 oder 18, **gekennzeichnet durch** einen Verkettungskreis (34), welcher ein von der Kamera (10) erzeugte Gesamtbild oder Teilbild mit dem Kontrastbild verkettet, insbesondere multipliziert.

20. Vorrichtung nach einem der Ansprüche 17 bis 19, **gekennzeichnet durch** ein Anzeigegerät (42), **durch** welches wahlweise das Gesamtbild, das Kontrastbild oder ein verkettetes Gesamtbild dargestellt werden können.

**Claims**

1. Method for evaluating fluorescence partial images of differing wavelength which are acquired by irradiation of the same point of an object by exciting light, wherein from the two partial images a quotient image is generated by pixel- by-pixel dividing of the pixel intensities of the two partial images, and for the quotient image a distribution curve is determined which specifies with which frequency a defined quotient occurs, **characterised in that**, depending on the width and/or the mean value of the distribution curve, a threshold value is determined, and **in that** a diagnostic image is generated from the quotient image, in which the pixels of the quotient image are variably modified depending upon how their intensity lies relative to the threshold value, as a result of which a contrast image is obtained.

2. Method according to Claim 1, **characterised in that** pixels of the quotient image are amplified in intensity, in particular are to maximum intensity, and/or are set a first colour if their intensity is greater than the threshold value, and are attenuated in intensity, in particular are set to minimum intensity, and/or are set to a second colour if their intensity is lower than the threshold value.

3. Method according to Claim 1 or 2, **characterised in that** in each instance a constant is added to the pixel signals of the two partial images before the formation of the quotient.

4. Method according to Claim 3, **characterised in that** the ratio of the two constants is equal to the ratio of the mean values of the pixel intensities of the two partial images.

5. Method according to Claim 4, **characterised in that** the same constant is added to the pixel signals of both partial images.

6. Method according to Claim 3, 4 or 5, **characterised in that** the constant added to the pixel signals of the partial images is chosen in a manner depending on the signal-to-noise ratio of the partial images.

7. Method according to Claim 6, **characterised in that** in the case of excitation of the fluorescence with blue light or UV light the first partial image is generated in the red and the second partial image is generated in the green and the constant to be added amounts to between 30 and 120, preferentially between 50 and 100, again preferentially approximately 80.

8. Method according to one of Claims 1 to 7, **characterised in that** the width of the distribution curve of the quotient image is determined in such a manner that an analytic distribution function is adapted to the distribution curve, in particular by a least-squares fit, and the width of the distribution curve and/or the maximum thereof and/or the mean value thereof is/are predetermined by the corresponding values of the analytic curve.

9. Method according to Claim 8, **characterised in that** the analytic distribution function is a Gaussian function.

10. Method according to one of Claims 1 to 9, **characterised in that** the threshold value is computed in a manner depending on the mean value of the distribution curve.

11. Method according to Claim 10, **characterised in that** regions of the distribution curve for which the frequency falls below a predetermined minimum value are left out of consideration.

12. Method according to Claim 11, **characterised in that** the mean value of the distribution curve is computed taking into account those regions of the curve in which the amplitude is greater than a predetermined fraction of the maximum of the distribution curve.

13. Method according to Claim 12, **characterised in that** the predetermined fraction amounts to between 0.05 and 0.3, preferentially between 0.1 and 0.2, again preferentially approximately 0.15.

14. Method according to one of Claims 10 to 13, **characterised in that** the threshold value is determined from the mean value of the distribution curve and from the width of the distribution curve multiplied by a constant.

15. Method according to Claim 14, **characterised in that** the constant amounts to between 2 and 5, preferentially approximately 3.5.

16. Method according to one of Claims 1 to 15, **characterised in that** a non-filtered overall image or a partial image of the object is concatenated with the contrast image, in particular is multiplied thereby.

17. Device for implementing the method according to one of Claims 1 to 16, having a camera (10) which provides at least two partial images of differing colour of an object (18), a computing circuit (20) which generates a quotient image from the two partial images by dividing, pixel by pixel, the pixel signals of the first partial image by those of the second partial image, and an analyser (24) which generates a distribution curve of the quotient image which represents the frequency of the occurrence of pixels with a predetermined colour ratio in the quotient image, the analyser (24) determines the maximum and/or the width of this distribution curve, and in that a contrast-image computing circuit (30) forms the pixels of a diagnostic image using at least those pixels of the quotient image which exceed a predetermined threshold value.

18. Device according to Claim 17, **characterised in that** the camera (10) includes a CCD or CMOS image-converter (14R, 14G, 14B) for generating RGB partial images for the three colours red, green and blue.

19. Device according to Claim 17 or 18, **characterised by** a concatenating circuit (34) which concatenates an overall image or a partial image generated by the camera (10) with the contrast image, in particular multiplies it thereby.

20. Device according to one of Claims 17 to 19, **characterised by** an indicating instrument (42) by which optionally the overall image, the contrast image or a concatenated overall image can be represented.

**Revendications**

1. Procédé d'évaluation d'image partielles de fluorescence de longueur d'onde différente qui sont obtenues du même endroit d'un objet en exposant à une lumière d'excitation, une image quotient étant produite à partir des deux images partielles par division pixel par pixel des intensités de pixels des deux images partielles et une courbe de distribution étant déterminée pour l'image quotient, qui indique à quelle fréquence un quotient défini survient, **caractérisé en ce qu'**une valeur seuil est déterminée en fonction de la largeur et/ou de la moyenne de la courbe de distribution et qu'une image de diagnostic est produite à partir de l'image quotient, dans laquelle les pixels de l'image quotient sont modifiés différemment selon la manière dont leur intensité se situe par rapport à la valeur seuil, de sorte que l'on obtient une image contrastée.

2. Procédé selon la revendication 1, **caractérisé en ce que** les pixels de l'image quotient sont renforcés dans leur intensité, en particulier réglés à l'intensité maximale et/ou à une première couleur, si leur intensité est supérieure à la valeur seuil et affaiblis dans leur intensité, en particulier réglés à l'intensité minimale et/ou à une deuxième couleur, si leur intensité est inférieure à la valeur seuil.

3. Procédé selon la revendication 1 ou 2, **caractérisé en ce qu'**une constante est ajoutée aux signaux de pixels de chacune des deux images partielles avant la formation de quotient.

4. Procédé selon la revendication 3, **caractérisé en ce que** le rapport des deux constantes est égal au rapport des valeurs moyennes des intensités de pixels des deux images partielles.

5. Procédé selon la revendication 4, **caractérisé en ce que** la même constante est ajoutée aux signaux de pixels des deux images partielles.

6. Procédé selon la revendication 3, 4 ou 5, **caractérisé en ce que** la constante ajoutée aux signaux de pixels des images partielles est choisie en fonction du rapport signal/bruit des images partielles.

7. Procédé selon la revendication 6, **caractérisé en ce qu'**en cas d'excitation de la fluorescence avec de la lumière bleue ou de la lumière UV, la première image partielle est produite dans le rouge et la deuxième image partielle dans le vert, et la constante ajoutée est comprise entre 30 et 120, de préférence entre 50 et 100, encore plus préférentiellement à peu près égale à 80.

8. Procédé selon une des revendications 1 à 7, **caractérisé en ce que** la largeur de la courbe de distribution de l'image quotient est déterminée de façon qu'une fonction de distribution analytique soit adaptée à la courbe de distribution, en particulier par une adaptation par les moindres carrés, et la largeur de la courbe de distribution et/ou son maximum et/ou sa valeur moyenne sont données par les valeurs correspondantes de la courbe analytique.

9. Procédé selon la revendication 8, **caractérisé en ce que** la fonction de distribution analytique est une fonction de Gauss.

10. Procédé selon une des revendications 1 à 9, **caractérisé en ce que** la valeur seuil est calculée en fonction de la valeur moyenne de la courbe de distribution.

11. Procédé selon la revendication 10, **caractérisé en ce qu'**on ne tient pas compte des régions de la courbe de distribution dans lesquelles la fréquence est inférieure à une valeur minimale prédéfinie.

12. Procédé selon la revendication 11, **caractérisé en ce que** la valeur moyenne de la courbe de distribution est calculée en tenant compte des régions de la courbe dans lesquelles l'amplitude est supérieure à une fraction prédéfinie du maximum de la courbe de distribution.

13. Procédé selon la revendication 12, **caractérisé en ce que** la fraction prédéfinie est comprise entre 0,05 et 0,3, de préférence entre 0,1 et 0,2, encore plus préférentiellement à peu près égale à 0,15.

14. Procédé selon une des revendications 10 à 13, **caractérisé en ce que** la valeur seuil est déterminée à partir de la valeur moyenne de la courbe de distribution et de la largeur de la courbe de distribution multipliée par une constante.

15. Procédé selon la revendication 14, **caractérisé en ce que** la constante est comprise entre 2 et 5, de préférence à peu près égale à 3,5.

16. Procédé selon une des revendications 1 à 15, **caractérisé en ce qu'**une image totale non filtrée ou une image partielle de l'objet est composée, en particulier multipliée avec l'image contrastée.

17. Dispositif pour réaliser le procédé selon une des revendications 1 à 16, qui présente une caméra (10), laquelle fournit au moins deux images partielles de couleur différente d'un objet (18), un circuit de calcul (20), lequel produit à partir des deux images partielles une image quotient en divisant pixel par pixel les signaux de pixels de la première image partielle par ceux de la deuxième image partielle, et un analyseur (24), lequel produit une courbe de distribution de l'image quotient, laquelle représente la fréquence de survenue de pixels ayant un rapport de couleur prédéfini dans l'image quotient, **caractérisé en ce que** l'analyseur (24) détermine le maximum et/ou la largeur de cette courbe de distribution, et que un circuit de calcul d'image contrastée (30) forme les pixels d'une image de diagnostic en utilisant au moins les pixels de l'image quotient qui dépassent une valeur seuil prédéfinie.

**18.** Dispositif selon la revendication 17, **caractérisé en ce que** la caméra (10) comprend un convertisseur d'image CCD ou CMOS (14R, 14G, 14B) pour produire des images partielles RVB pour les trois couleurs rouge, vert et bleu.

**19.** Dispositif selon la revendication 17 ou 18, **caractérisé par** un circuit de composition (34), lequel compose, en particulier multiplie, une image totale ou une image partielle produite par la caméra (10) avec l'image contrastée.

**20.** Dispositif selon une des revendications 17 à 19, **caractérisé par** un appareil d'affichage (42), au moyen duquel au choix l'image totale, l'image contrastée ou une image totale composée peut être représentée.

20  22  24  26  28  30  32  34

a   s        b   D

14R  14G  14B

18

12

16R  16G  16B

10

44  a b s  42

38

46  40  36

Fig.1

EP 2 120 682 B1

12

Fig. 2

Fig. 3

Fig. 4

Fig. 5

EP 2 120 682 B1

Fig. 6

Insel·100

Fig. 7

**EP 2 120 682 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004491 A1
- DE 102004024494 A1
- DE 10207918 A1